(19) Europäisches Patentamt
European Patent Office
Office européen des brevets

(11) **EP 4 316 336 B1**

(12) **EUROPEAN PATENT SPECIFICATION**

(45) Date of publication and mention
of the grant of the patent:
**29.07.2026  Bulletin 2026/31**

(21) Application number: **22212545.2**

(22) Date of filing: **09.12.2022**

(51) International Patent Classification (IPC):
*A61B 1/00* (2006.01)   *H02K 11/00* (2016.01)
*H02K 23/02* (2006.01)   *H02K 44/04* (2006.01)
*H02K 99/00* (2014.01)   *A61M 25/00* (2006.01)
*A61M 25/01* (2006.01)   *A61B 17/22* (2006.01)
*A61B 17/3207* (2006.01)   *A61B 34/00* (2016.01)

(52) Cooperative Patent Classification (CPC):
**H02K 99/20; A61B 1/00158; A61B 17/320758;**
**A61B 34/73; A61M 25/0127; H02K 11/0094;**
**H02K 23/02; H02K 44/04;** A61B 17/22;
A61B 2017/00398; A61B 2034/2059;
A61B 2090/374; A61M 25/0009; A61M 25/0082;
A61M 2025/0166

(54) **DEVICE CONFIGURED TO BE INSERTED INTO A BLOOD VESSEL OF A HUMAN BEING AND/OR AN ANIMAL, AND ELECTRIC MOTOR AND MAGNETOHYDRODYNAMIC MODULE FOR THE DEVICE**

VORRICHTUNG ZUM EINSETZEN IN EIN BLUTGEFÄSS EINES MENSCHEN UND/ODER EINES TIERES SOWIE ELEKTROMOTOR UND MAGNETOHYDRODYNAMISCHES MODUL FÜR DIE VORRICHTUNG

DISPOSITIF CONÇU POUR ÊTRE INSÉRÉ DANS UN VAISSEAU SANGUIN D'UN ÊTRE HUMAIN ET/OU D'UN ANIMAL, ET MOTEUR ÉLECTRIQUE ET MODULE MAGNÉTOHYDRODYNAMIQUE POUR LE DISPOSITIF

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.08.2022  US 202217816774**
**02.08.2022  DE 202022104403 U**
**02.08.2022  US 202217816779**
**02.08.2022  DE 202022104405 U**
**02.08.2022  LU 502623**

(43) Date of publication of application:
**07.02.2024  Bulletin 2024/06**

(73) Proprietor: **Max-Planck-Gesellschaft zur Förderung der Wissenschaften e.V.**
**80539 München (DE)**

(72) Inventors:
• **Sitti, Metin**
**Tübingen (DE)**
• **Phelan, Martin**
**Tübingen (DE)**

(74) Representative: **Pearl Cohen EU**
**Nibelungenstraße 84**
**80639 München (DE)**

(56) References cited:
EP-B1- 2 012 699     KR-B1- 100 971 013
US-A1- 2018 221 656     US-B1- 6 594 517

• **NGUYEN KIM TIEN ET AL: "Guide-Wired Helical Microrobot for Percutaneous Revascularization in Chronic Total Occlusion in-Vivo Validation", IEEE TRANSACTIONS ON BIOMEDICAL ENGINEERING, IEEE, USA, vol. 68, no. 8, 22 December 2020 (2020-12-22), pages 2490 - 2498, XP011866638, ISSN: 0018-9294, [retrieved on 20210715], DOI: 10.1109/TBME.2020.3046513**

- **THOMAS STANLEY GREGORY ET AL: "The Magnetohydrodynamic Effect and Its Associated Material Designs for Biomedical Applications: A State-of-the-Art Review", ADVANCED FUNCTIONAL MATERIALS, WILEY - V C H VERLAG GMBH & CO. KGAA, DE, vol. 26, no. 22, 24 February 2016 (2016-02-24), pages 3942 - 3952, XP072412676, ISSN: 1616-301X, DOI: 10.1002/ADFM.201504198**

**Description**

The present invention is directed to an electric motor configured to be integrated into a tip of a device configured to be inserted into a blood vessel of a human being and/or an animal, a magnetohydrodynamic module configured to be integrated into a tip of a device configured to be inserted into a blood vessel of a human being and/or an animal, and a device, optionally a catheter, comprising the electric motor and the magnetohydrodynamic module.

Any discussion of the related art throughout the specification should in no way be considered as an admission that such related art is widely known or forms part of common general knowledge in the field.

Wherever definitions of terms used in this description are given, the respective description is just one possible specific definition out of many possible definitions of the respective term and is thus not intended to limit the scope of the disclosure to this specific definition.

EP 2 012 699 B1 is directed to a device for treatment of human or animal tissue by means of a movable instrument. The device comprises a means for generating a magnetic stator field in which the tissue is located, in particular a magnetic resonance tomograph. At least one conductor loop is provided on or in the instrument, and a generator for generating a current that flows through the conductor loop, in order to generate a magnetic useful field which, in conjunction with the stator field, generates a resulting action for applying a useful force to the instrument.

US 2018/0221656 A1 is directed to a device exploiting magneto-hydrodynamics (MHD) for localized delivery of material into a target or extraction of material from a target. The device includes a frame comprising a space for conductive fluid and the material, at least one pair of electrodes facing each other, a source of electric current, a magnet, and an opening. The electric current and the magnetic field are synchronized so that the material can be moved from the volume between the electrodes through the opening towards the target or from the target through the opening towards the volume. According to US 2018/0221656 A1 the volume is ≤2000 mm3, in proviso that mean distance between tips of the electrodes is ≤20 mm.

Cardiovascular disease (CVD) is the leading cause of death worldwide according to the World Health Organization, responsible for 17.9 million deaths as of 2019. One of the most common complications in CVDs known as Deep Vein Thrombosis (DVT) refers to the formation of a blood clot within the deep vein of the patient. This is highly dangerous because the clot formation can break off and travel through the bloodstream to the lung, which might cause a pulmonary embolism (PE). If this clot is large enough, blood flow to the lungs could be blocked leading to death. This can also result in long-term damage known as post-thrombotic syndrome. PE is the third leading cause of death within CVDs in the United States, after myocardial infarction and stroke. For high-risk PE in which thrombolysis is contraindicated due to high risks of bleeding or patients are in critical condition and cannot wait for intravenous fibrinolytics, mechanical thrombectomy is usually recommended.

Mechanical thrombectomy includes fragmentation in which a mechanical device such as a balloon or motorized elements are used to break up thrombi in pulmonary arteries.

Increased contact surfaces of fragments can improve the effectiveness of thrombolysis. However, catheter-based aspiration can be integrated to reduce the risk of distal embolization caused by the fragments. Aspiration involves placing a catheter or sheath in the pulmonary arteries and removing the clot using a balloon or external vacuum pump by creating negative pressure. However, this technique is constrained by the catheter size in relation to the size and volume of the clots. Known systems are often not successful due to its limited maneuverability in the right vein and pulmonary arteries.

Arterial stenosis is also a dangerous disease caused by calcified deposits and plaque that form within the arteries to restrict blood flow. This complication, also known as atherosclerosis, is known as peripheral artery disease and directly influences blood flow to the legs and feet. Mechanical removal of such depots exists commercially, but known systems are limited by their high-frequency drilling speeds which can cause complications such as blood vessel wall damage from heat or microcavitation.

Tissue reperfusion is often the most important factor determining the success of the occlusion removal procedure. Restoration of blood flow to the diseased area determined the viability of organ functionality. In terms of thrombectomy, these devices are created to ensure the most rapid and complete reperfusion of tissue. However, tissue reperfusion injury or ischemia-reperfusion injury (IRI), which is when blood supply to the tissue is restored after a period of oxygen-deprivation (hypoxia), can occur in the heart, lungs, kidney, gut, skeletal muscle, and brain, and can lead to multi-system organ failure. Some possible methods to prevent IRI include ischaemic preconditioning in which brief and repetitive restoration episodes are introduced to control the severity of tissue damage and post-conditioning in which rapid interruption of flow is controlled during the beginning moments of reperfusion.

In the above-mentioned fragmentation method catheter drillers may be used. Conventional catheter drillers require an external power source to transmit torque down the shaft of a flexible device. However, this limits the steerability of the device when the device needs to be soft enough to reduce blunt force trauma to the vessel and flexible to steer through tortuous vessels. Additionally, conventional catheter drillers require higher torque output to transmit torque to the distal end of the device.

Conventional catheter drillers also do not exist for magnetic resonance imaging (MRI), which provides excellent soft tissue visualization. Conventional commercial drillers are composed of ferromagnetic materials, limiting their usage to imaging modalities outside of MRI such as fluoroscopy. These modalities rely upon ionizing radiation, which is deemed unsafe for extended procedures. Similarly in the literature, other catheter drillers incorporate rotating magnetics controlled by an external robotic arm to cause torque control. However, also these instruments are limited to fluoroscopic/ultrasound guidance for visualization.

There is a growing need for MRI-compatible surgical instruments in cardiovascular interventions such as treating arterial thrombosis/stenosis.

Against the background of this prior art, one task of the present disclosure may be to disclose a method and/or an apparatus which are each suitable for enriching the prior art.

One concrete object of the disclosure might be formulated as to provide a MRI-compatible thrombectomy device.

The task is solved by the features of the independent claim. The dependent claims have optional further developments of the disclosure as their content.

Accordingly, the task is solved by a device configured to be inserted into a blood vessel of a human being and/or an animal, wherein the device comprises an electric motor being integrated into a tip of the device, wherein the electric motor consists of non-magnetic material and is configured to be driven by an external magnetic field, and wherein the electric motor comprises a shaft configured to supply torque generated by the electric motor to an actuator of the device. The device comprises a magnetohydrodynamic module being integrated into the tip of the device, wherein the magnetohydrodynamic module comprises at least two electrodes arranged to propel an electrically conducting fluid by applying a voltage to the at least two electrodes and/or measuring a voltage applied to the at least two electrodes by the electrically conducting fluid streaming between the at least two electrodes. The device comprises a control unit configured to measure the voltage applied to the at least two electrodes by the electrically conducting fluid streaming between the at least two electrodes and control the torque produced by the electric motor and supplied to the actuator based on the measured voltage.

In other words, the electric motor is configured to be integrated into a tip of a device configured to be inserted into a blood vessel of a human being and/or an animal, wherein the electric motor consists of non-magnetic material and is configured to be driven by an external, optionally static, magnetic field.

The term "consisting of" is exhaustive, i.e., the electric motor, and optionally also the device or at least that part of the device configured to be inserted into the blood vessel, does not contain magnetic material.

In other words, the electric motor, and optionally also the device or at least that part of the device configured to be inserted into the blood vessel, are MRI-compatible.

Magnetic resonance imaging (MRI) is a medical imaging technique used in radiology to form pictures of the anatomy and/or the physiological processes of the body. MRI scanners use strong magnetic fields, magnetic field gradients, and radio waves to generate images, optionally of the organs in the body of a human being and/or an animal. MRI does not involve X-rays or the use of ionizing radiation, which distinguishes it from CT and PET scans. MRI is a medical application of nuclear magnetic resonance (NMR) which can also be used for imaging in other NMR applications, such as NMR spectroscopy.

Non-magnetic material is any material that does not meet the definition of a magnetic material according to this disclosure. A magnetic material according to this disclosure is any material that may produce its own persistent magnetic field even in the absence of an applied magnetic field. Those materials are called magnets. Moreover, a magnetic material is any material that produces a magnetic field in response to an applied external magnetic field - a phenomenon known as magnetism - wherein the produced magnetic field is considerable in the context of or destroys MRI compatibility of the electric motor. More specifically, there are several types of magnetism, and all materials exhibit at least one of them. However, in this disclosure the term magnetism refers to ferromagnetic and ferrimagnetic materials only. These materials are the only ones that can retain magnetization and become magnets. Ferrimagnetic materials, which include ferrites and the magnetic materials magnetite and lodestone, are similar to but weaker than ferromagnetics. In other words, in this disclosure paramagnetic materials (such as platinum, aluminum, and oxygen) are considered non-magnetic material. Moreover, in this disclosure diamagnetic materials (such as carbon, copper, water, and plastic) which are repelled by both poles and which are compared to paramagnetic and ferromagnetic substances even more weakly repelled by a magnet are also considered non-magnetic materials.

The above-described electric motor provides several advantages. One of these advantages is that the electric motor may be integrated into the tip of the device without compromising the device's MRI compatibility.

The electric motor may be configured to be driven by the external magnetic field produced by a medical device, optionally a medical imaging device, further optionally an MRI-device.

This provides the advantage that the external magnetic field that is acting on the device anyway during imaging may be used not only for imaging but also for driving the electric motor resulting in a decreased number of parts needed for the electric motor and enabling a compact design of the electric motor as well as the tip of the device.

The external magnetic field may have a field strength between 0,05 T and 25 T, optionally between 0,1 T and 21 T, further optionally between 0,1 T and 9,4 T, further optionally between 1,5 T and 7,0 T, further optionally between 0,1 T and 3,0 T.

In other words, the electric motor may be configured such that it may be driven with an external magnetic field having a field strength between 0,05 T and 25 T, optionally between 0,1 T and 21 T, further optionally between 0,1 T and 9,4 T, further optionally between 1,5 T and 7,0 T, further optionally between 0,1 T and 3,0 T.

The above-mentioned field strength are used for MRI devices. An electric motor being suitable to be driven by such field strength ensures MRI compatibility of the device.

The electric motor may comprise, optionally may consist of, a brushed DC motor and/or a brushless motor.

The brushed DC (direct current) electric motor may be an internally commutated electric motor designed to be run from a direct current power source and utilizing one or more electric brushes for contact.

A brushed DC motor provides the advantage that it may be driven with relatively low control effort, i.e., a torque generated by the electric motor may be varied by varying the power supply to the electric motor. This results in a simple and reliable solution that may be achieved with a relatively low number of needed parts.

The brushless motor may be an AC induction motor, wherein said motor uses the external magnetic field, e.g., provided or produced by the MRI device, as its stator. The rotor of the brushless motor may comprise of multipolar coils arranged in such a manner to enable continuous rotation by supplying alternating current to the coils depending upon the orientation of the coils over time. Therefore, a non-magnetic encoder may be provided to track an orientation or position of each one of the coils, respectively, and a controller connected to the encoder and configured to control a current supplied to each coil based on the respective orientation of the coil measured by the encoder. The controller may be part of the electric motor, i.e., may be integrated into the tip, or may be an external part configured to be connected to the electric motor, more specifically to the encoder thereof. The brushless motor may use a slip ring instead of a commutator.

The electric motor may comprise a rotor with at least one, optionally two or three, coils configured to produce a magnetic field when being supplied with electrical energy. Different shapes of the rotor are possible.

The coils may comprise, optionally consist of, copper and/or any other electrically conductive non-magnetic material.

In other words, the electric motor may comprise, irrespective if it is brushed or brushless, a rotor configured to be driven by the external magnetic field. The rotor may comprise one, two, three or more coils powered with electric current thereby generating a magnetic field that changes when the rotor rotates. The external magnetic field may be a static magnetic field, i.e., acting as a stator, causing the powered rotor to rotate due to the Lorentz force.

This provides the advantage that such static magnetic fields are produced by MRI devices such that MRI compatibility of the device, especially the electric motor, is ensured.

The electric motor comprises a shaft configured to supply torque generated by the electric motor to an actuator of the device.

The actuator may be any kind of medical tool requiring rotational actuation. The actuator may be part of the tip of the device, i.e., may be integrated into the tip of the device. More specifically, the actuator of the device may comprise, optionally consist of, a receptacle connected to a drive shaft of the electric motor and configured to receive a drill bit (i.e., the actuator may comprise or may consist of a drilling device or a rotational drilling mechanism) configured to remove an occlusion, such as a thrombus, in the blood vessel when being supplied with torque from the electric motor via the drive shaft, i.e., when be driven by the electric motor. Additionally or alternatively, the actuator of the device may comprise, optionally consist of, a cutting tool configured to cut tissue (optionally of the animal and/or the human being) when being supplied with torque from the electric motor, i.e., when be driven by the electric motor. Additionally or alternatively, the actuator of the device may be configured to release an additional (medical) device into the blood vessel (such as a stent) when being supplied with torque from the electric motor, i.e., when be driven by the electric motor.

The electric motor may not comprise a stator, i.e., may be built without stator. As already indicated above, the external magnetic field, optionally produced by an MRI device, may act as the stator, i.e., may replace the stator.

The above description of the electric motor may be summarized in other words with respect to a more concrete implementation of the disclosure as outlined in the following, wherein the following is not to be understood to limit the scope of the disclosure as defined by the claims, but is merely described for exemplary purposes.

With the above electric motor a novel MRI-driven, Lorentz-force based direct current electric motor for mechanical thrombectomy is disclosed. The motor may operate similarly to a direct current brush motor, however, the stator (which is typically a permanent magnetic housing), may be replaced by the magnetic field of the MRI. This field strength ranges from 1.5 to 7 Tesla (T) for clinical scanners. This allows for direct torque transmission on the catheter tip without influencing the rest of the soft body of the catheter. The motor may also transmit a high torque output for removing occlusions effectively.

The rotor of the MRI-driven direct current electric motor may comprise three coils wherein each coil may be powered sequentially to allow for continuous and maximum torque generation, analogous to a DC brush motor. This electromagnetic torque generation phenomena may work at maximum torque output according to the following equations:

$$\tau_{out} = m \: x \: B_0$$

$$\tau_{out} = m_1 \, B_0 cos\omega t\beta + m_2 B_0 \sin\left(\omega t + \frac{2\pi}{3}\right)\beta + m_3 B_0 \sin\left(\omega t + \frac{2\pi}{3}\right)\beta$$

$$\beta = \sin\alpha + \cos\alpha$$

$$m_1 = m_2 = m_3 = NIAB_0$$

$$\tau_{out} = NIAB_0(cos\omega t + 2\sin\left(\omega t + \frac{2\pi}{3}\right)\beta)$$

Where N is the number of coil loops, I represents the current, A is the area of each coil loop, and $B_0$ is the external magnetic field of the MRI. This design is very simple and thus easy to implement, compact and reliable.

Alternatively an MRI-driven direct current electromagnetic induction motor may be provided. Here, a multi-coil (e.g., three sets of coils located opposite to one another; analogous to a standard DC electromagnetic induction motor) module may be driven using the external field as with the above described MRI-driven direct current electric motor but without the use of a commutator. Instead, to ensure continuous rotation, the precise orientation of each coil module may be measured using a non-magnetic encoder. By knowing the angle of each coil module, a current to each coil can be adjusted properly for improved power efficiency and safety over DC brushed motors.

Regardless of the used type of motor, the motor may drive a drill bit of the catheter.

The advantages of the Lorentz force-based driller may be - inter alia - a direct torque transmission for clot removal, a flexibility for steering through tortuous vessels, MRI-compatibility, a strong output torque due to the strength of the external MRI field, a highly scalable design for manufacturing, and/or the fact that no external, MRI-compatible capital equipment is needed (like air pressure control) as used with many known devices.

Furthermore, the disclosure is directed to a magnetohydrodynamic module configured to be integrated into a tip of a device configured to be inserted into a blood vessel of a human being and/or an animal. The magnetohydrodynamic module comprises at least two electrodes arranged to propel an electrically conducting fluid by applying a voltage to the at least two electrodes and/or measuring a voltage applied to the at least two electrodes by the electrically conducting fluid streaming between the at least two electrodes.

The above given description with respect to the electric motor applies mutatis mutandis to the magnetohydrodynamic module and vice versa.

Magnetohydrodynamics (MHD; also called magneto-fluid dynamics or hydro-magnetics) is the study of the magnetic properties and behavior of electrically conducting materials or electrically conducting fluids. Examples of such magneto-fluids include plasmas, liquid metals, salt water, and electrolytes. Also blood and anticoagulants or thrombolytic agents are electrically conducting fluids.

The magnetohydrodynamic module may comprise a permanent magnet and/or a coil being configured to produce or producing a magnetic field, and/or may be driven by an external magnetic field, e.g., produced by an **MRI** device. Especially in the latter case, but not limited thereto, the magnetohydrodynamic module may consist of non-magnetic material, e.g. copper and/or aluminum. This would ensure **MRI** compatibility of the magnetohydrodynamic module.

The term "arranged to propel" may mean that dimensions of the two electrodes, e.g., a distance of the electrodes to each other and a size of the electrodes, are chosen such that the electrically conducting fluid may be propelled by applying the known or predefined voltage to the two electrodes. When the voltage is applied to the two electrodes an electric field is generated, especially between the two electrodes, which causes the electrically conducting fluid together with the magnetic field to flow, i.e., to be propelled.

The electrically conducting fluid which may be propelled by the two electrodes may be located inside and/or outside the magnetohydrodynamic module and/or the device.

The magnetohydrodynamic module may have the form of capsule that may be added to the tip of the device.

The above-described magnetohydrodynamic module provides several advantages. One of these advantages is that it might be integrated into the tip of the device itself - similarly to the above-described electric motor, and thus does not negatively affect other parts of the device, such as a soft body of a catheter in case the device is a catheter.

The magnetohydrodynamic module may comprise an opening for removing debris being transported by the electrically conducting fluid propelled by the at least two electrodes.

More specifically, the electrically conducting fluid may be blood located in the blood vessel in which the tip of the device is inserted. The blood may be propelled such that a blood flow towards an opening in the device is generated to allow the blood transporting or containing the debris to enter together with the debris into the device. Thereby the debris may be transported through the device, e.g., a soft body of a catheter, out of the human or animal body.

This provides the advantage that the magnetohydrodynamic module acts as an aspiration module.

The magnetohydrodynamic module may comprise, additionally or alternatively, an opening for propelling the electrically conducting fluid into the blood vessel.

In this case the magnetohydrodynamic module may act a pump for bringing, e.g., a anticoagulants or thrombolytic agents thereby actively assisting in removing a thrombus in the blood vessel.

The above description of the magnetohydrodynamic module may be summarized in other words with respect to a more concrete implementation of the disclosure as outlined in the following, wherein the following is not to be understood to limit the scope of the disclosure as defined by the claims, but is merely described for exemplary purposes.

An MRI-driven magnetohydrodynamic aspiration module may be provided. For this proposed scheme, a capsule can be placed on the end of a catheter with electrodes that may be located - depending of the orientation of the tip of the device with respect to the MRI magnetic field - perpendicular to the B field vector. When a voltage is applied across both electrodes, a resultant pressure force is generated to propel an electrically conducting fluid between and around the electrodes to cause flow. Such flow can be controlled, e.g., to redirect heat from coils of the electric motor to minimize thermal damage to the electric motor and/or blood vessel(s), remove debris from an area in front of the tip, e.g., an area of drilling, to be passed back through the catheter, and/or propel anticoagulants and/or thrombolytic agents into the occlusion to assist in removal/tissue reperfusion.

Additionally or alternatively, the electrodes can also be used as an MRI-driven, magneto flow meter to measure the blood flow pressure at the catheter tip. This measurement is orientation dependent but can be adjusted accordingly if the precise orientation of the catheter tip is known. Therefore, by adjusting an actuation of an actuator of the device, such as a rotational speed of a drill bit, simultaneously while measuring blood pressure flow, the device can safely monitor and control the reperfusion of local tissue to reduce the chances of IRI.

The advantages of the magnetohydrodynamic module may be - inter alia - to provide an easy way to integrate aspiration module and/or a module that might be manufactured at low cost and/or highly scalable, and/or that may be MRI-compatible. Additionally or alternatively, the magnetohydrodynamic module may allow direct pressure control to assist in tissue reperfusion, non-magnetic, scalable pressure sensing and/or direct flow control for temperature regulation.

Furthermore, the disclosure is directed to a device configured to be inserted into a blood vessel of a human being and/or an animal. The device comprises the above-described electric motor and/or the above-described magnetohydrodynamic module being integrated into the tip of the device.

In other words, both, the electric motor and the magnetohydrodynamic module, may be integrated into the tip of the device.

The above given description with respect to the electric motor and the magnetohydrodynamic module applies mutatis mutandis to the device and vice versa.

The device may be MRI compatible, i.e., the device, optionally the tip thereof and/or the part of the device being configured to be inserted into the blood vessel may consist of non-magnetic material.

The device comprises a control unit configured to measure the voltage applied to the at least two electrodes by the electrically conducting fluid streaming between the at least two electrodes and control the torque produced by the electric motor and supplied to the actuator based on the measured voltage.

The device may comprise a catheter, optionally an endovascular catheter, and the tip in which electric motor and/or the magnetohydrodynamic module is/are integrated may be a tip of the catheter.

The tip may be located at a distal end of the device, i.e., that end of the device which is inserted into the blood vessel.

The actuator may be, i.e., consist of, or comprise a driller configured to remove an occlusion in the blood vessel when being supplied with torque from the electric motor.

The device may comprise a soft body being connected to the tip of the device. The device and optionally the tip and/or the soft body thereof may comprise a working channel extending through the tip and/or the soft body.

The device, especially the tip of the device, may be steerable. Therefore, the tip may comprise a set of coils surrounding the tip. The device may comprise power wires arranged to supply the set of coils with electrical energy.

The set of coils may comprise four side coils arranged around the tip such that a straight line standing orthogonal on a longitudinal direction of the tip crosses a center of the respective side coil, i.e., the four side coils are arranged around the tip such that a magnetic flux direction of the four side coils points towards a center line of the tip.

A first and a second one of the side coils may be connected in series. Additionally or alternatively, a third and a fourth one of the side coils may be connected in series. Additionally or alternatively, the first and the second one of the side coils may be arranged on opposite sides of the tip. Additionally or alternatively, the third and the fourth one of the side coils may be arranged on opposite sides of the tip and in-between the first and the second one of the side coils.

A turn number of at least one of the four side coils may be between 2 and 40 (i.e, including 2 and 40). Optionally, the turn number of at least one of the four side coils may be between 4 and 30 (i.e, including 4 and 30). Optionally, the turn number of at least one of the four side coils may be 7.

The set of coils may comprise at least one axial coil arranged around the tip such that a straight line extending in parallel to the longitudinal direction of the tip crosses a center of the at least one axial coil.

At least one of the coils of the set of coils may be manufactured using laser machining, laser lithography and/or manually wound.

At least one of the coils of the set of coils may have, an optionally rectangular, Archimedean spiral coil design.

At least one of the coils of the set of coils may have an in-plane design.

The four side coils may be arranged on the same, optionally flexible, circuit board.

Provided may be a first method for controlling a movement of the above-described device, optionally the tip thereof, in a magnetic field, optionally the one produced by the MRI device,.

The first method may comprise determining a torque that needs to be applied onto the device such that the device carries out the movement.

The first method may comprise determining a minimum current that needs to be supplied to each coil of the set of coils, respectively, to reach the determined torque by solving an optimization problem.

The first method may comprise supplying the determined minimum current to each coil of the set of coils, respectively, such that the device carries out the movement.

The optimization problem (for determining the minimum current) may be defined as follows:

$$\mathbf{I}^{*} = \arg\min_{\mathbf{I}} ||\tau_{\text{coils}} - \tau_{\text{des}}||^{2} + \alpha||\mathbf{I}||_{\mathbf{R}}^{2}$$

wherein:

- I may represent a current supplied to each coil of the set of coils, respectively,
- $\tau$_coils may represent a total torque generated by the set of coils when being supplied with the current I,
- $\tau$_des may represent the torque that needs to be applied onto the device such that the device carries out the movement, and
- R may represent a resistance of each coil of the set of coils.

Determining the torque may comprise solving a further optimization problem to minimize the torque to be applied onto the device such that the device carries out the movement.

The device may comprise a flexible substantially rod-shaped portion (i.e., the soft body of the device and/or the tip of the device). The movement may comprise a deformation of said rod-shaped portion resulting in a movement of a tip of said rod-shaped portion (i.e., the tip of the device).

Solving the further optimization problem may comprise determining the deformation of said rod-shaped portion that is needed for the movement of the tip of said rod-shaped portion from an actual location to a desired location such that a torque that is required for the deformation of said rod-shaped portion is minimized. Additionally or alternatively, solving the further optimization problem may comprise determining the torque that needs to be applied onto the catheter such that the device carries out the movement to be equal to the torque that is required for the deformation of said rod-shaped portion.

The deformation that is needed for the movement of the tip of said rod-shaped portion from the actual location to the desired location may be determined using a model, optionally a Cosserat model, depicting the nonlinear dynamics of said rod-shaped portion.

The method may comprise receiving user input with respect to the movement via an user interface, optionally comprising a joystick, of the device.

The method may comprise determining an actual position of the device, optionally the tip thereof, using medical imaging (e.g., MRI). Additionally or alternatively, at least one of the above-described methods may comprise an automated controlling of the movement based on the determined actual position.

The method may comprise displaying an actual position of the device, optionally the tip thereof, and/or a position of the device, optionally the tip thereof, after carrying out the movement on a display device. The display device may be connected to the device and/or a medical imaging device (e.g., the MRI device).

At least one of the above described methods may comprise displaying the actual position of the device and/or the position of the device after carrying out the movement with respect to a tissue, optionally of the human being and/or the animal, on the display device.

The magnetic field may be produced by a medical imaging device, optionally a magnetic resonance imaging device, and/or the magnetic field may be a static magnetic field.

Additionally or alternatively, a data processing device may be provided which is configured to carry out the above-described method at least partly.

Additionally or alternatively, a computer program may be provided, wherein the computer program may comprise instructions which, when the program is executed by a computer, e.g., the data processing device, cause the computer to carry out the above-described method at least partly.

Additionally or alternatively, a computer-readable (storage) medium may be provided, wherein the computer-readable medium comprises instructions which, when executed by a computer, cause the computer to carry out the above-described method at least partly.

The term "method" as used herein may include a computer-implemented method. The expression "computer-implemented method" covers claims which involve computers, computer networks or other programmable apparatus, whereby at least one feature is realised by means of a program. A computer-implemented method may be a method which is at least partly carried out by a data processing unit, e.g. a computer.

The term "controlling" may be defined as a process in a system in which one or more variables as input variables influence other variables as output variables due to the laws peculiar to the system. Additionally or alternatively, the term "controlling" may be defined as a process in which a variable, the controlled variable (the variable to be controlled), is continuously recorded, compared with another variable, the reference variable, and influenced in the sense of an adjustment to the reference variable.

The term "movement" may include any displacement or change of a position of a device, here optionally the medical device, optionally the tip thereof. In one possible interpretation the movement may be a motion. A motion may be the phenomenon in which an object, here the medical device, optionally the tip thereof, changes its position with respect to space and time, optionally the magnetic field.

The term "determining" may include carrying out one or more mathematical operations in order to determine based on a given input in a given manner a desired output.

The term "torque" may be the rotational equivalent of a linear force. The term "torque" may be also referred to as the moment, moment of force, rotational force or turning effect. The torque may represent the capability of a force to produce change in the rotational motion of a body, such as the rode shaped portion of the medical devie. The torque may be defined as the product of the magnitude of the force and the perpendicular distance of the line of action of the force from the axis of rotation. In three dimensions, the torque may be a pseudovector; for point particles, it is given by the cross product of the position vector (distance vector) and the force vector. The magnitude of torque of a rigid body may depend on three quantities: the force applied, the lever arm vector connecting the point about which the torque is being measured to the point of force application, and the angle between the force and lever arm vectors. The toque may be defined by the force that is applied to the tip of the medical device.

Instead of the term "current", the term "electric current" may be used. An electric current is a stream of charged particles, such as electrons or ions, moving through an electrical conductor or space. It is measured as the net rate of flow of electric charge through a surface or into a control volume. The moving particles are called charge carriers, which may be one of several types of particles, depending on the conductor. In electric circuits the charge carriers are often electrons moving through a wire. In semiconductors they can be electrons or holes. In an electrolyte the charge carriers are ions, while in plasma, an ionized gas, they are ions and electrons. The SI unit of electric current is the ampere, or amp, which is the flow of electric charge across a surface at the rate of one coulomb per second. Electric currents create magnetic fields, which may be used to actuate or move the medical device in the (external) magnetic field. In ordinary conductors, they cause Joule heating.

Instead of the term "coil", the term electromagnetic coil may be used. An electromagnetic coil may be an electrical conductor such as a wire in the shape of a coil, spiral or helix. An electric current may be passed through the wire of the coil to generate a magnetic field. A current through any conductor creates a circular magnetic field around the conductor due to Ampere's law. One advantage of using the coil shape may be that it increases the strength of the magnetic field produced by a given current. The magnetic fields generated by the separate turns of wire all pass through the center of the coil and add (superpose) to produce a strong field there. The more turns of wire, the stronger the field produced may be and the stronger the effect of Joule heating may be.

An optimization problem may be described as the problem of finding substantially the best solution from all feasible solutions.

The device, especially the catheter, may be a medical device. A medical device may be any device intended to be used for medical purposes. According to one possible definition a medical device may be an instrument, apparatus, implement, machine, contrivance, implant, in vitro reagent, or other similar or related article, including a component part, or accessory which is intended for use in the diagnosis of disease or other conditions, or in the cure, mitigation, treatment, or prevention of disease, in man or other animals, and/or intended to affect the structure or any function of the body of man or other animals, and which does not achieve its primary intended purposes through chemical action within or on the body of man or other animals and which is not dependent upon being metabolized for the achievement of its primary intended purposes. The term "medical device" may or may not include software functions. According to another possible definition the term "medical device" may mean any instrument, apparatus, appliance, software, material or other article, whether used alone or in combination, including the software intended by its manufacturer to be used specifically for diagnostic and/or therapeutic purposes and necessary for its proper application, intended by the manufacturer to be used for human beings or animals for the purpose of diagnosis, prevention, monitoring, treatment or alleviation of disease, diagnosis, monitoring, treatment, alleviation of or compensation for an injury or handicap, investigation, replacement or modification of the anatomy or of a physiological process, and/or control of conception, and which does not achieve its principal intended action in or on the human and/or animal body by pharmacological, immunological or metabolic means, but which may be assisted in its function by such means.

Instead of the term "resistance", the term "electrical resistance" may be used. The resisatnce multiplied by the current may be equal to the (electrical) voltage.

A rod-shaped portion may be a portion or part of the medical device that may have a substantially circular cross section. The rod-shaped portion may have cylindrical form wherein a height or length of the rod-shaped portion exceeds the diamter of the rod-shaped portion. The tip of the rod-shaped portion may be outermost part of the rod-shaped portion in a forward direction of the medical device. The tip of the rod-shaped portion may comprise a region around the circumference at or near the end of the outermost part of the rod-shaped portion. The rod-shaped portion may comprise or may be realized using a tube. The tip may be called insertion tip of the medical device.

The Cosserat model may be based on the Cosserat rod theory. This approach may allow for a substantially exact solution to the static of a continuum robot, as it is not subject to any assumption. It solves a set of equilibrium equations between position, orientation, internal force and torque of the robot, here the medical device, optionally the rod-shaped portion thereof. Creating an accurate model that can predict the shape of a continuum robot allows to properly control the robot's shape.

A catheter may comprise a thin tube, i.e., the rod shaped portion, made from medical grade materials serving a broad range of functions. Catheters are medical devices that can be inserted in the body to treat diseases and/or perform a surgical procedure. By modifying the material or adjusting the way catheters are manufactured, it is possible to tailor catheters for cardiovascular, urological, gastrointestinal, neurovascular, and ophthalmic applications. The process of inserting a catheter is "catheterization". A catheter may comprise a thin, flexible tube ("soft" catheter) through catheters are available in varying levels of stiffness depending on the application. This may be taken into account by the (Cosserat) model. The catheter may be configured to be inserted into a body cavity, duct, or vessel, brain, skin or adipose tissue. Functionally, the catheter may allow drainage, administration of fluids or gases, access by surgical instruments, and/or perform a wide variety of other tasks depending on the type of catheter. The catheter may be a so called probe used in preclinical or clinical research for sampling of lipophilic and hydrophilic compounds, protein-bound and unbound drugs, neurotransmitters, peptides and proteins, antibodies, nanoparticles and nanocarriers, enzymes and vesicles.

An endovascular catheter may be a catheter configured to be used for endovascular aneurysm repair (EVAR) which is a type of minimally-invasive endovascular surgery used to treat pathology of the aorta, most commonly an abdominal aortic aneurysm (AAA). When used to treat thoracic aortic disease, the procedure is then specifically termed TEVAR for "thoracic endovascular aortic/aneurysm repair." The procedure may involve the placement of an expandable stent graft within the aorta to treat aortic disease without operating directly on the aorta.

A medical imaging device may be a device configured to be used for medical imaging. Medical imaging is the technique and process of imaging the interior of a body. Medical imaging may incorporate radiology, which uses the imaging technologies of X-ray radiography, magnetic resonance imaging, ultrasound, endoscopy, elastography, tactile imaging, thermography, medical photography, and/or nuclear medicine functional imaging techniques as positron emission tomography (PET) and single-photon emission computed tomography (SPECT).

A magnetic resonance imaging device is a medical imaging device configured to be used in magnetic resonance imaging (MRI) which is a medical imaging technique that may be used in radiology to form pictures of the anatomy and the physiological processes of the body. MRI scanners use strong magnetic fields, magnetic field gradients, and radio waves to generate images of the organs in the body.

A working channel may be a channel, optionally having a circular shape form, arranged in the rod-shaped portion of the medial device. The working channel may extend throughout the whole rod-shaped portion. The working channel may be used to transport a (medical) device, a gas and/or a fluid from one end of the medical device, optionally located outside of the body, to the tip of the rod-shaped portion. The working channel may house or accommodate devices permanently and/or temporarily, such as a camera, an eletrical wire and/or a light source, at least partly.

A driller may be the device that may be equipment with or comprise a drill (bit), wherein the drill may comprise one or more sharp surfaces that are configured to cut through (human and/or animal) tissue, optionally a thrombus. The drill may thus be a cutting tool. The drill may have a substantially circular cross-section. The drill may be driven via the electric motor such that the drill rotates and cuts through the thrombus which thereby might decompose in multiple smaller parts, i.e., the so-called debris. In other words, the driller may comprise or consist of a drill bit directly connected to the motor or a receptacle for the drill bit that is configured to connect the drill bit to the drive shaft of the electric motor. However, the disclosure is in no way limited to a driller and other configurations could encompass other surgical tools than a driller which require rotation to operate and provide treatment. Therefore, when the term driller is used in the disclosure this should be understood that this is just one specific example for the actuator and that, additionally or alternatively, other tolls besides a driller may be used in connection with the electric motor and/or the hydromagnetic module.

A thrombus (plural thrombi), colloquially called a blood clot, is the final product of the blood coagulation step in hemostasis. There are two components to a thrombus: aggregated platelets and red blood cells that form a plug, and a mesh of cross-linked fibrin protein. The substance making up a thrombus is sometimes called cruor. A thrombus is a healthy response to injury intended to stop and prevent further bleeding, but can be harmful in thrombosis, when a clot obstructs blood flow through healthy blood vessels in the circulatory system. In the microcirculation consisting of the very small and smallest blood vessels the capillaries, tiny thrombi known as microclots can obstruct the flow of blood in the capillaries. This can cause a number of problems particularly affecting the alveoli in the lungs of the respiratory system resulting from reduced oxygen supply. Microclots have been found to be a characteristic feature in severe cases of COVID-19, and in long COVID. Mural thrombi are thrombi that adhere to the wall of a large blood vessel or heart chamber. They are most commonly found in the aorta, the largest artery in the body, more often in the descending aorta, and less often in the aortic arch or abdominal aorta. They can restrict blood flow but usually do not block it entirely. They appear grey-red with alternating light and dark lines (known as lines of Zahn) which represent bands of white blood cells and red blood cells (darker) entrapped in layers of fibrin. All the above described forms of thrombi may be subsumed under the term "thrombus" used in this disclosure.

An actuator according to the present disclosure may be or may comprise a driller, but may in general be every device that may be actuated by applying torque (from the electric moor) to the actuator.

The term electric motor may be understood as a motor that may be driven - inter alia - by supplying electrical power or energy to the motor.

Wherever the term "is configured to" in this disclosure is used, the term should be interpreted in a manner limiting the respective device which is configured to be used or to do something in that the device is structurally suitable or adapted to be used for the specific purpose. For example, in the present disclosure the device, especially the tip thereof, the electric motor, the actuator, especially the driller, and the magnetohydrodynamic module are configured to be inserted into a blood vessel of an animal and/or a human being, respectively and in combination. Therefore, all these parts have dimensions that are limited by the dimensions, optionally an inner diameter, of the blood vessel of the human being and/or the animal in which they should be inserted. To be configured to be inserted into the blood vessel the device, (optionally the catheter, optionally the soft body and/or the tip of the catheter,) the electric motor, the actuator (optionally the driller, further optionally the drill (bit) of the driller) and/or the magnetohydrodynamic module may have an outer diameter of 0,1 mm to 10 mm, 0,5 mm - 6 mm, 1,5 mm to 5 mm, 2 mm to 3 mm, and/or 2,5 mm or 2 mm. To be configured to be inserted into the blood vessel the device (optionally the catheter, optional the soft body and/or the tip of the catheter), the electric motor, the actuator (optionally the driller, further optionally the drill (bit) of the driller) and/or the magnetohydrodynamic module may have a length of 1 mm to 40 mm and/or 50 mm, 1 mm, 5 mm and/or 10 mm to 30 mm, 15 mm, 20 mm, and/or 25 mm. The same is true for the term "configured to be integrated into the tip", i.e., an electric motor, an actuator and/or a magnetohydrodynamic module having the above described dimensions is/are configured to be integrated into the tip of the device. Integrated into the tip may mean completely or at least partly integrated.

The term "integrated into the tip" may mean that the electric motor and/or the magnetohydrodynamic module form(s), optionally together with other parts such as a lamp, the above-described coils for steering the device, the above described actuator (e.g., the driller) and so on, one part of the device, i.e., the tip thereof. The term "integrated into the tip" of the device may mean that the device comprises one housing accommodating one or both of the electric motor and the magnetohydrodynamic module. However, it may also be possible that the magnetohydrodynamic module is provided in a separate housing that may be fixed to the rest of the tip accommodating the electric motor, or vice versa. However, in both cases the tip may be one, optionally multifunctional, part that may be connected to and separated from the rest of the device. With respect to a catheter that may mean that the catheter may be divided substantially into two parts, i.e., a soft body and the tip which is as such a separate part from the soft body but may be connected to a distal end of the soft body. The tip may be connected permanently or detachably/removably to the rest of the device; in case of the catheter to the soft body thereof. The connection between the tip and the rest of the device may be for example a screw fitting or bolted connection. In other words, the term "integrated into the tip" may mean in its broadest sense that not a juxtaposition of separate but functionally interacting individual components is provided but an assembly of parts is provided which forms the tip or the distal end of the device.

Wherever the expressions "for example", "by way of example", "such as", "e.g." and the like are used, this shall be construed as being followed by the expression "and without limitation", unless expressly stated otherwise. Similarly, "an example," "exemplary," and the like shall be construed as not limiting or as a non-exhaustive list.

For numerical indications, they are to be understood as both conclusive and non-exhaustive, i.e., for example, "a device" is to be understood as "at least one device and/or exactly one device".

The term "substantially" allows for variations that do not adversely affect the intended purpose. Descriptive terms shall be understood to be modified by the term "substantially" even if the term "substantially" is not expressly stated.

The terms "comprising" and "including" and "with" and "having" (and similarly "comprises," and "includes," and "has") and the like are used interchangeably and have the same meaning.

Consequently, unless the context clearly or explicitly requires otherwise, the words "comprising," "including," and the like in the description and claims are to be understood in an inclusive sense and not in an exclusive or exhaustive sense, i.e., in the sense of "including but not limited to."

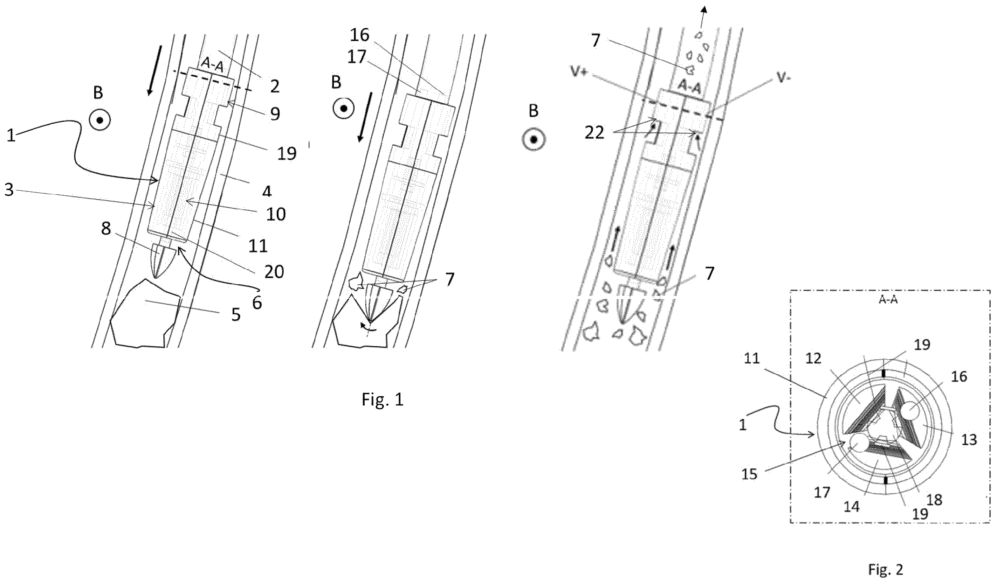

Hereinafter, an embodiment of disclosure will be described with reference to figures 1 and 2.

Fig. 1    schematically shows a perspective view on a catheter in three different states of removing a thrombus in a blood vessel, wherein an electric motor and a magnetohydrodynamic module are both integrated into a tip of the catheter, and

Fig. 2    schematically shows a cross-sectional view of a part of the tip of the catheter of figure 1 where the electric motor is located.

As required, a detailed embodiment is disclosed herein; however, it is to be understood that the disclosed embodiment is merely exemplary of the disclosure that may be embodied in various forms. The figures are not necessarily to scale, and some features may be exaggerated to show details of particular components. Therefore, specific structural and functional details disclosed in the figures are not to be interpreted as limiting, but merely as a representative basis for teaching one skilled in the art to variously employ the present disclosure.

Throughout the figures the same components are indicated by the same reference signs. More specifically, in the following a catheter 1 according to the disclosure will be described in detail with reference to figures 1 and 2, wherein the same components are labelled with the same reference signs throughout the single views of the figures.

As can be gathered from figure 1, the catheter 1 comprises a soft body 2 and a tip 3 forming a distal end of the catheter 1. The catheter 1 is shown in a state in all three views of figure 1, in which the catheter 1, i.e., the tip 3 and the distal part of the soft body 2 being connected to the tip 3, is inserted into a blood vessel 4 of a human being and/or an animal in a magnetic field B produced by a (not shown) MRI device, wherein the magnetic field B is aligned perpendicular to a direction of movement of the catheter 1 along the blood vessel 4 (as indicated by the arrow in figure 1).

The first state shown in figure 1 on the left, is a state where the tip 3 of the catheter 1 approaches a blockage 5, such as a thrombus, being located in the blood vessel 4 and clocking the blood vessel 4. The second state - following the first state - shown in figure 1 in the middle, is a state where the tip 3 of the catheter 1 reaches the blockage 5 and an electric motor 10 of or integrated into the tip 3 is powered such that a drilling operation of a driller 6 of the tip 3 starts in order to break or remove the blockage 5. The third state - following the second state - shown in figure 1 on the right, is a state where the blockage 5 is (already) broken by a drill bit 8 of the driller 6 and a debris 7 of the blockage 5 is removed using a magnetohydrodynamic (aspiration) module 9 of or integrated into the tip 3, wherein the magnetohydrodynamic module 9 is located between the electric motor 10 being located at the distal end of the tip 3 and the soft body 2.

More specifically, the tip 3 comprises the electric motor 10, the magnetohydrodynamic module 9 and the driller 6. All these parts may be accommodated into one housing 11; the housing 11 may be for example manufactured by injection molding. All parts of the tip 3 and the soft-body 2 are MRI compatible, i.e., all parts consist of non-magnetic material. The housing 11 is shown partly transparent such that the electric motor 10, the magnetohydrodynamic module 9 and the driller 6 may be seen as indicated with the dotted lines in figure 1.

The electric motor 10 in the example shown in figures 1 and 2 is a brushed DC motor, wherein the electric motor 10 does not comprise a stator itself; instead the MRI device producing the static magnetic field B is acting as the stator for the electric motor 10 such that the electric motor 10 may be driven by the (with respect to the catheter 1 and especially the tip 3 thereof) external magnetic field B produced by the MRI device. The magnetic field B may have a field strength between 0,05 T and 25 T, optionally between 0,1 T and 21 T, further optionally between 0,1 T and 9,4 T, further optionally between 1,5 T and 7,0 T, further optionally between 0,1 T and 3,0 T.

As can be gathered partly from figure 1 but in detail from figure 2 which is a perspective sectional view of the tip 3 at the location indicated by A-A in figure 1, the electric motor 10 comprises a rotor 15 with three coils 12, 13, 14 each one comprising copper windings for producing a magnetic field when being supplied with electrical energy, respectively. The electric motor 10 is connected to a ground wire 16 and a terminal wire 17 for supplying direct current (DC) to the electric motor 10, and comprises a commutator 18 (i.e., a rotary electrical switch that periodically reverses the current direction in the coils 12 - 14) as well as brushes 19 (e.g., made out of non-magnetic metallic material such as carbon) for making sliding contact with successive segments of the commutator 18 as it rotates. The housing 11 may comprise a silver coating 19 on its inner diameter. The electric motor 10 comprises a shaft 20 connected to the rotor 15 and to a (not shown) drill retainer portion of the driller 6 in which the drill bit 8 is inserted such that the torque generated by the rotor 15 of electric motor 10 is supplied to the drill bit 8 resulting in a rotational movement of the drill bit 8. By rotating the drill bit 8 the blockage 5 may be removed by breaking the blockage 5 into pieces, the so-called debris 7, as can be gathered from the view in the middle of figure 1 and the view on the right side of figure 1.

To get the debris 7 out of the blood vessel 4, the magnetohydrodynamic module 9 may be powered. The magnetohydrodynamic module 9 has two electrodes, here the ground wire 16 and the terminal wire 17 that are arranged to have a gap in between where an electric field is formed when a voltage (indicated by V- and V+ view on the right side of figure 1) is applied onto the two wires 16, 17, i.e., when the electric motor 10 is driven or powered. Since the two electrodes or wires 16, 17 are spaced apart from each other and the electric field is formed between them, blood as an electrically conducting fluid is propelled in the blood vessel 5 such that a blood flow towards opening(s) 21 of the magnetohydrodynamic module 9 is generated, wherein the opening(s) 21 are arranged such that the blood flow and thus the debris 7 can enter the soft body 2 of the catheter 1 through the opening(s) 21 to be transported through the soft body 3 outside of the body of the human being and/or animal to whom the blood vessel 4 belongs to.

In the above example the two wires 16, 17 were used as electrodes for the module 9. However, it would also be possible to provide separate electrodes for the module 9.

Furthermore, the above described aspiration function is just one out of a variety of functions of the magnetohydrodynamic module 9. The magnetohydrodynamic module 9 may for example also be used to propel fluid from the soft body 3 into the blood vessel 4 through dedicated openings in the module 9. Additionally or alternatively, the magnetohydrodynamic module 9 may be used to measure a voltage applied to the two electrodes, the voltage being caused by a blood flow streaming between the two electrodes. The measured voltage may be used as an input for a (not shown) control unit, the control unit being configured to control a power supply to the electric motor 10 thereby controlling the driller 6.

Moreover, in the above example a brushed DC motor was used as the electric motor 10. Additionally or alternatively, a brushless motor as defined in the introductory part of this description may be used as the electric motor 10.

## REFERENCE SIGNS

|    |    |
|----|----|
| 1  | (medical) device, optionally catheter |
| 2  | soft body |
| 3  | tip |
| 4  | blood vessel |
| 5  | occlusion/blockage |
| 6  | actuator, optionally driller |
| 7  | debris |
| 8  | drill bit |
| 9  | magnetohydrodynamic module |
| 10 | electric motor |
| 11 | housing |
| 12 | first coil |
| 13 | second coil |
| 14 | third coil |
| 15 | rotor |
| 16 | ground wire |
| 17 | terminal wire |
| 18 | commutator |
| 19 | silver coating |
| 20 | shaft |
| 21 | opening(s) |

| B | magnetic field |

## Claims

1. Device (1) configured to be inserted into a blood vessel (4) of a human being and/or an animal, wherein the device (1) comprises:

   - an electric motor (10) being integrated into a tip (3) of the device (1), wherein the electric motor (10) consists of non-magnetic material and is configured to be driven by an external magnetic field (B), and wherein the electric motor (10) comprises a shaft configured to supply torque generated by the electric motor (10) to an actuator (6) of the device (1), and
   - a magnetohydrodynamic module (9) being integrated into the tip (3) of the device (1), wherein the magnetohydrodynamic module (9) comprises at least two electrodes arranged to propel an electrically conducting fluid by applying a voltage to the at least two electrodes and/or measuring a voltage applied to the at least two electrodes by the electrically conducting fluid streaming between the at least two electrodes,

**characterized in that** the device (1) comprises a control unit configured to measure the voltage applied to the at least two electrodes by the electrically conducting fluid streaming between the at least two electrodes and control the torque produced by the electric motor (10) and supplied to the actuator (6) based on the measured voltage.

2. Device (1) according to claim 1, **characterized in that** the device (1) comprises a catheter and the tip (3) in which electric motor (10) and/or the magnetohydrodynamic module (9) is/are integrated is a tip of the catheter.

3. Device (1) according to any of claim 1 or 2, **characterized in that** the tip (3) is located at a distal end of the device (1).

4. Device (1) according to any of claims 1 to 3, **characterized in that** the actuator (6) is or comprises a driller configured to remove an occlusion (5) in the blood vessel when being supplied with torque from the electric motor (10).

5. Device (1) according to any of claims 1 to 4, **characterized in that** the electric motor (10) is configured to be driven by the external magnetic field (B) produced by an MRI-device.

6. Device (1) according to any of claims 1 to 5, **characterized in that** the external magnetic field (B) has a field strength between 0,05 T and 25 T, optionally between 0,1 T and 21 T, further optionally between 0,1 T and 9,4 T, further optionally between 1,5 T and 7,0 T, further optionally between 0,1 T and 3,0 T.

7. Device (1) according to any of claims 1 to 6, **characterized in that** the electric motor (10) comprises a brushed DC motor and/or a brushless motor.

8. Device (1) according to any of claims 1 to 7, **characterized in that** the electric motor (10) comprises a rotor (15) with at least one, optionally two or three, coils (12 - 14) configured to produce a magnetic field when being supplied with electrical energy.

9. Device (1) according to any of claims 1 to 8, **characterized in that** the electric motor (10) does not comprise a stator.

10. Device (1) according to any of claims 1 to 9, **characterized in that** the magnetohydrodynamic module (9) comprises an opening (21) for removing debris (7) being transported by the electrically conducting fluid propelled by the at least two electrodes.

11. Device (1) according to any of claims 1 to 10, **characterized in that** the magnetohydrodynamic module (9) comprises an opening (21) for propelling the electrically conducting fluid into the blood vessel (4).

**Patentansprüche**

1. Vorrichtung (1), die zum Einführen in ein Blutgefäß (4) eines Menschen und/oder eines Tieres ausgebildet ist, wobei die Vorrichtung (1) umfasst:

- einen Elektromotor (10), der in eine Spitze (3) der Vorrichtung (1) integriert ist, wobei der Elektromotor (10) aus nichtmagnetischem Material besteht und so ausgelegt ist, dass er durch ein externes Magnetfeld (B) angetrieben wird, und wobei der Elektromotor (10) eine Welle umfasst, die so ausgelegt ist, dass sie das vom Elektromotor (10) erzeugte Drehmoment an einen Aktuator (6) der Vorrichtung (1) überträgt, und
- ein magnetohydrodynamisches Modul (9), das in die Spitze (3) der Vorrichtung (1) integriert ist, wobei das magnetohydrodynamische Modul (9) mindestens zwei Elektroden umfasst, die so angeordnet sind, dass sie ein elektrisch leitendes Fluid antreiben, indem eine Spannung an die mindestens zwei Elektroden angelegt wird und/oder eine Spannung gemessen wird, die an die mindestens zwei Elektroden durch das zwischen den mindestens zwei Elektroden strömende elektrisch leitende Fluid angelegt wird,

**dadurch gekennzeichnet, dass** die Vorrichtung (1) eine Steuereinheit umfasst, die so konfiguriert ist, dass sie die an den mindestens zwei Elektroden durch das zwischen den mindestens zwei Elektroden strömende elektrisch leitende Fluid angelegte Spannung misst und das vom Elektromotor (10) erzeugte und an den Aktuator (6) gelieferte Drehmoment auf der Grundlage der gemessenen Spannung steuert.

**2.** Vorrichtung (1) nach Anspruch 1, **dadurch gekennzeichnet, dass** die Vorrichtung (1) einen Katheter umfasst und die Spitze (3), in die der Elektromotor (10) und/oder das magnetohydrodynamische Modul (9) integriert ist/sind, eine Spitze des Katheters ist.

**3.** Vorrichtung (1) nach einem der Ansprüche 1 oder 2, **dadurch gekennzeichnet, dass** sich die Spitze (3) an einem distalen Ende der Vorrichtung (1) befindet.

**4.** Vorrichtung (1) nach einem der Ansprüche 1 bis 3, **dadurch gekennzeichnet, dass** der Aktuator (6) ein Bohrer ist oder umfasst, der so konfiguriert ist, dass er eine Okklusion (5) im Blutgefäß entfernt, wenn er vom Elektromotor (10) mit Drehmoment versorgt wird.

**5.** Vorrichtung (1) nach einem der Ansprüche 1 bis 4, **dadurch gekennzeichnet, dass** der Elektromotor (10) so ausgelegt ist, dass er durch das von einem MRT-Gerät erzeugte externe Magnetfeld (B) angetrieben wird.

**6.** Vorrichtung (1) nach einem der Ansprüche 1 bis 5, **dadurch gekennzeichnet, dass** das externe Magnetfeld (B) eine Feldstärke zwischen 0,05 T und 25 T aufweist, gegebenenfalls zwischen 0,1 T und 21 T, ferner gegebenenfalls zwischen 0,1 T und 9,4 T, ferner gegebenenfalls zwischen 1,5 T und 7,0 T, ferner gegebenenfalls zwischen 0,1 T und 3,0 T.

**7.** Vorrichtung (1) nach einem der Ansprüche 1 bis 6, **dadurch gekennzeichnet, dass** der Elektromotor (10) einen bürstenbehafteten Gleichstrommotor und/oder einen bürstenlosen Motor umfasst.

**8.** Vorrichtung (1) nach einem der Ansprüche 1 bis 7, **dadurch gekennzeichnet, dass** der Elektromotor (10) einen Rotor (15) mit mindestens einer, gegebenenfalls zwei oder drei Spulen (12 - 14) umfasst, die so ausgebildet sind, dass sie bei Zufuhr elektrischer Energie ein Magnetfeld erzeugen.

**9.** Vorrichtung (1) nach einem der Ansprüche 1 bis 8, **dadurch gekennzeichnet, dass** der Elektromotor (10) keinen Stator umfasst.

**10.** Vorrichtung (1) nach einem der Ansprüche 1 bis 9, **dadurch gekennzeichnet, dass** das magnetohydrodynamische Modul (9) eine Öffnung (21) zum Entfernen von Schmutzpartikeln (7) aufweist, die von der durch die mindestens zwei Elektroden angetriebenen elektrisch leitenden Flüssigkeit mitgeführt werden.

**11.** Vorrichtung (1) nach einem der Ansprüche 1 bis 10, **dadurch gekennzeichnet, dass** das magnetohydrodynamische Modul (9) eine Öffnung (21) zum Ausstoßen der elektrisch leitenden Flüssigkeit in das Blutgefäß (4) aufweist.

**Revendications**

**1.** Dispositif (1) conçu pour être inséré dans un vaisseau sanguin (4) d'un être humain et/ou d'un animal, dans lequel le dispositif (1) comprend :

- un moteur électrique (10) intégré dans une pointe (3) du dispositif (1), dans lequel le moteur électrique (10) est constitué d'un matériau non magnétique et est conçu pour être entraîné par un champ magnétique externe (B), et dans lequel le moteur électrique (10) comprend un arbre conçu pour fournir un couple généré par le moteur électrique (10) à un actionneur (6) du dispositif (1), et
- un module magnétohydrodynamique (9) intégré dans la pointe (3) du dispositif (1), dans lequel le module magnétohydrodynamique (9) comprend au moins deux électrodes agencées pour propulser un fluide conducteur de l'électricité par application d'une tension aux au moins deux électrodes et/ou par mesure d'une tension appliquée aux au moins deux électrodes par le fluide conducteur de l'électricité s'écoulant entre les au moins deux électrodes,

**caractérisé en ce que** le dispositif (1) comprend une unité de commande conçue pour mesurer la tension appliquée aux au moins deux électrodes par le fluide conducteur de l'électricité s'écoulant entre les au moins deux électrodes et pour commander le couple produit par le moteur électrique (10) et fourni à l'actionneur (6) sur la base de la tension mesurée.

**2.** Dispositif (1) selon la revendication 1, **caractérisé en ce que** le dispositif (1) comprend un cathéter et la pointe (3) dans laquelle le moteur électrique (10) et/ou le module magnétohydrodynamique (9) est/sont intégré(s) est une pointe du cathéter.

3.  Dispositif (1) selon l'une quelconque des revendications 1 ou 2, **caractérisé en ce que** la pointe (3) est située au niveau d'une extrémité distale du dispositif (1).

4.  Dispositif (1) selon l'une quelconque des revendications 1 à 3, **caractérisé en ce que** l'actionneur (6) est ou comprend un outil de forage conçu pour éliminer une occlusion (5) dans le vaisseau sanguin lorsqu'il est alimenté en couple par le moteur électrique (10).

5.  Dispositif (1) selon l'une quelconque des revendications 1 à 4, **caractérisé en ce que** le moteur électrique (10) est conçu pour être entraîné par le champ magnétique externe (B) produit par un dispositif d'IRM.

6.  Dispositif (1) selon l'une quelconque des revendications 1 à 5, **caractérisé en ce que** le champ magnétique externe (B) a une intensité de champ comprise entre 0,05 T et 25 T, éventuellement entre 0,1 T et 21 T, éventuellement en outre entre 0,1 T et 9,4 T, éventuellement en outre entre 1,5 T et 7,0 T, éventuellement en outre entre 0,1 T et 3,0 T.

7.  Dispositif (1) selon l'une quelconque des revendications 1 à 6, **caractérisé en ce que** le moteur électrique (10) comprend un moteur à courant continu (CC) à balais et/ou un moteur sans balais.

8.  Dispositif (1) selon l'une quelconque des revendications 1 à 7, **caractérisé en ce que** le moteur électrique (10) comprend un rotor (15) doté d'au moins une, éventuellement de deux ou trois, bobines (12 - 14) conçues pour produire un champ magnétique lorsqu'elles sont alimentées en énergie électrique.

9.  Dispositif (1) selon l'une quelconque des revendications 1 à 8, **caractérisé en ce que** le moteur électrique (10) ne comprend pas de stator.

10. Dispositif (1) selon l'une quelconque des revendications 1 à 9, **caractérisé en ce que** le module magnétohydrodynamique (9) comprend une ouverture (21) pour éliminer les débris (7) transportés par le fluide conducteur de l'électricité propulsé par les au moins deux électrodes.

11. Dispositif (1) selon l'une quelconque des revendications 1 à 10, **caractérisé en ce que** le module magnétohydrodynamique (9) comprend une ouverture (21) pour propulser le fluide conducteur de l'électricité dans le vaisseau sanguin (4).

Fig. 2

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2012699 B1

- US 20180221656 A1